# EUROPEAN PATENT SPECIFICATION

(11) **EP 2 688 724 B1**
(45) Date of publication and mention of the grant of the patent: **18.11.2015**
(21) Application number: 12706098.6
(22) Date of filing: 14.02.2012
(51) Int. Cl.: B29C 45/16

(54) **MANUFACTURING METHOD AND PRODUCT**
HERSTELLUNGSVERFAHREN UND PRODUKT
PROCÉDÉ DE FABRICATION ET PRODUIT

(30) Priority: 23.03.2011 GB 201104842
(43) Date of publication of application: 29.01.2014
(73) Proprietor: DePuy (Ireland), Co Cork (IE)
(72) Inventor: BEEDALL, Duncan, Leeds West Yorkshire LS11 8DT (GB); REASON, Mark, Leeds West Yorkshire LS11 8DT (GB)
(74) Representative: Alton, Andrew
(86) International application number: PCT/GB2012/050317
(87) International publication number: WO 2012/127200

(56) References cited:
- WO-A1-2005/097239
- FR-A1- 2 386 411
- US-A1- 2004 155 171
- US-A1- 2010 180 400

## Description

The present invention relates to a manufacturing method and a product produced by the manufacturing method. In particular, the present invention relates to methods of making surgical instruments and prostheses.

Surgical instruments and prostheses are typically formed from a relatively small number of materials, which are selected for their properties, including biocompatibility, strength and resilience. Typical materials include metals such as stainless steel and plastics. The chosen materials, and the methods by which the instruments are formed, vary according to the particular function of the instrument or prosthesis.

Certain surgical instrument may be required to withstand significant forces which are generated during normal use, for instance a surgical impactor which is used to transfer an impaction force to an orthopaedic implant. It is clear that an impactor must be relatively strong to withstand the impaction force without damage. It is known to form such an impactor from a single block of plastic, for instance Radel® (Polyphenylsulfone) which has been machined to the required shape. Under high loads such a machined plastic component my fracture. Machining plastic is also a relatively expensive manufacturing method.

Alternatively, surgical instruments which are required to withstand high loads may be formed partly from plastic and partly from metal. However, while this may prove stronger than machined plastic, the high stress experienced during loading may cause the separate components to come apart due to vibration. Forming surgical instruments from multiple components, in particular where metal is used, is relatively expensive.

It is a requirement of reusable surgical instruments that they must be easy to clean. A machined plastic instrument may offer a relatively easy to clean solution as its exterior surface may be relatively smooth. Instruments formed from a combination of plastic and metal however may be relatively difficult to clean due to the interfaces between the components (or at least users may perceive the instrument as being difficult to clean).

WO 2005/097239 describes a high pressure medicinal dispenser and method of manufacture. The dispenser may have a handle made of hard plastic with a plurality of interior ribs with spaces between them and overmolded with soft rubber material which fills the spaces between the ribs and provides a grip.

US 2010/0180400 describes a handle for a medical device arid a method of fabricating the handle. The handle includes a core element, for example, a plastic core element, having an outer surface and raised indicia, such as, letters or logos, extending beyond the outer " surface. A light-transferring material, such as a translucent thermoplastic elastomer, can be over-molded to completely cover the raised indicia of the core element with the raised indicia visible through the light-transferring material.

US 2004/0155171 describes a lens and method for molding the lens without significant surface distortion. A cavity mold having a first core mold portion with strakes is used to create a first molded form having ribs proportioned and spaced apart to prevent distortions on one surface of the lens due to shrinkage. A second core mold portion replaces the first core mold portion for creation of a second molded form having complementary ribs which are similarly proportioned to prevent distortions in an opposite surface of the lens. The first form acts as a mold for the second form, the complementary ribs fuse together and form a homogeneous mass between the opposite surfaces of the lens.

It is an object of embodiments of the present invention to obviate or mitigate one or more of the problems associated with the prior art, whether identified herein or elsewhere.

According to a first aspect of the present invention there is provided a method of manufacturing a surgical instrument or prosthesis comprising: injecting a first material into a first mould to form an interim component, portions of the interim component defining at least one channel; and injecting the second material into a second mould containing at least part of the channel of the interim component so that the channel is filled with the second material, characterized in that the maximum wall thickness of the interim component is less than 5mm, and the maximum width of the at least one channel is less than 5mm, and wherein the first material and the second material have similar structural properties and comprise the same plastic.

An advantage of the first aspect of the present is that by producing a surgical instrument or prosthesis using two plastic injection moulding steps an instrument or prosthesis comprising a substantial thickness of plastic may be produced with a reduced risk of surface sinking or voids being left within the mould. Furthermore, the second injection moulding step may be arranged to produce an instrument with a smooth exterior surface, which is easy to clean. The instruments may also be cheaper than equivalent instruments manufactured through machining plastic or metal or coupling together multiple plastic or metal components.

The interim component may comprise at least two ribs each having a thickness less than 5mm, the at least one channel being defined between the ribs.

The interim component and the second mould may be arranged such that the maximum width of the cavity between the interim component and the mould wall is less than 5mm.

The first and second materials may be different colours.

At least one of the first and second materials may further comprise at least one additive.

The plastic may comprise a high or ultra high performance polymer.

According to a second aspect of the present invention there is provided an injection moulded surgical instrument or prosthesis comprising: a body, wherein the body is formed in a two shot injection moulding process using a first material and a second material respectively, the body comprising interlaced portions of said first and second materials characterized in that the body has a minimum thickness which exceeds 5mm and that the maximum thickness of any portion of the first material is less than 5mm and the maximum thickness of any portion of the second material is less than 5mm, and wherein the first material and the second material have similar structural properties and comprise the same plastic.

The present invention will now be described, by way of example only, with reference to the accompanying figures, in which:
Figures 1 and 2 illustrate front and rear views respectively of an interim component formed using a manufacturing method in accordance with a first embodiment of the present invention; and
Figures 3 and 4 illustrate front and rear views respectively of a surgical instrument formed using a manufacturing method in accordance with the first embodiment of the present invention.

It is known to manufacture products, including surgical instruments and prostheses, through injection moulding. A material such as thermoplastic or thermosetting plastic is heated and mixed to ensure a uniform consistency. The molten material is forced under pressure into a mould cavity where it cools and hardens. The amount of material required to fill the cavities of the mould is called a shot.

It is known that for a particular selected plastic material there is a maximum wall thickness (that is, the maximum thickness of any portion of the moulded article taking the shortest possible distance between exterior surfaces of the moulded article) that can be achieved before significant surface sinking or voids within the mould cavity occur. The maximum achievable wall thickness is dependent upon the material properties of the selected plastic and can be determined empirically for a given plastic. The skilled person will be able to readily determine though empirical testing the maximum wall thickness that can be achieved for a desired moulded article using any suitable plastic. A typical maximum thickness is 5mm. Although for certain materials the maximum could be larger, in the majority of applications 5mm is considered to be a sensible limit. Furthermore, the maximum thickness of different portions of a complex shape may vary. There are a number of "rules of thumb" which have been determined through empirical testing. For instance, to increase the strength of a thick wall of material, thinner ribs may be applied running perpendicular to the plane of the wall. To prevent sinking on the outside of the wall it is desirable to limit the thickness of the ribs to approximately 40% to 60% of the thickness of the wall.

It is known to use injection moulding to apply a layer of a plastic material over part or the whole of an existing interim component. This technique may be referred to as over-moulding or two shot moulding. One known application of this is to couple together two different forms of plastic having different material properties. For the example of a toothbrush, a first, stiff plastic may form the body of a handle, while a second, softer plastic forms a hand grip. Two shot moulding is also known for coating other materials such as metals with plastic, and for joining together two separate components.

Surgical instruments and prostheses, which may otherwise be suitable for manufacturing using injection moulding, may require a maximum wall thickness which exceeds the achievable limits for suitable plastics. Consequently, the use of injection moulding for surgical instruments and prostheses has been limited.

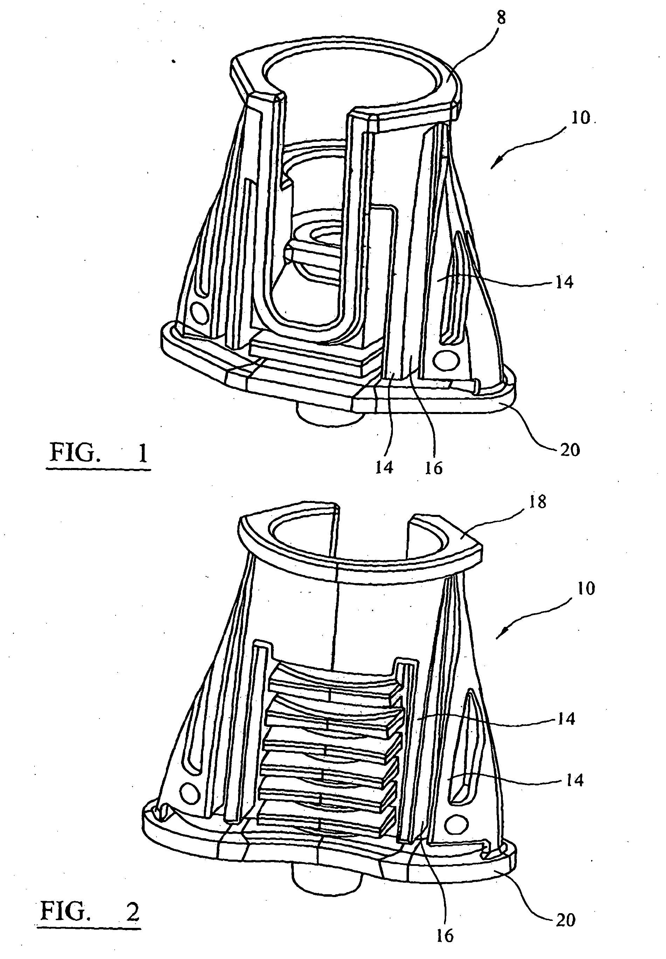

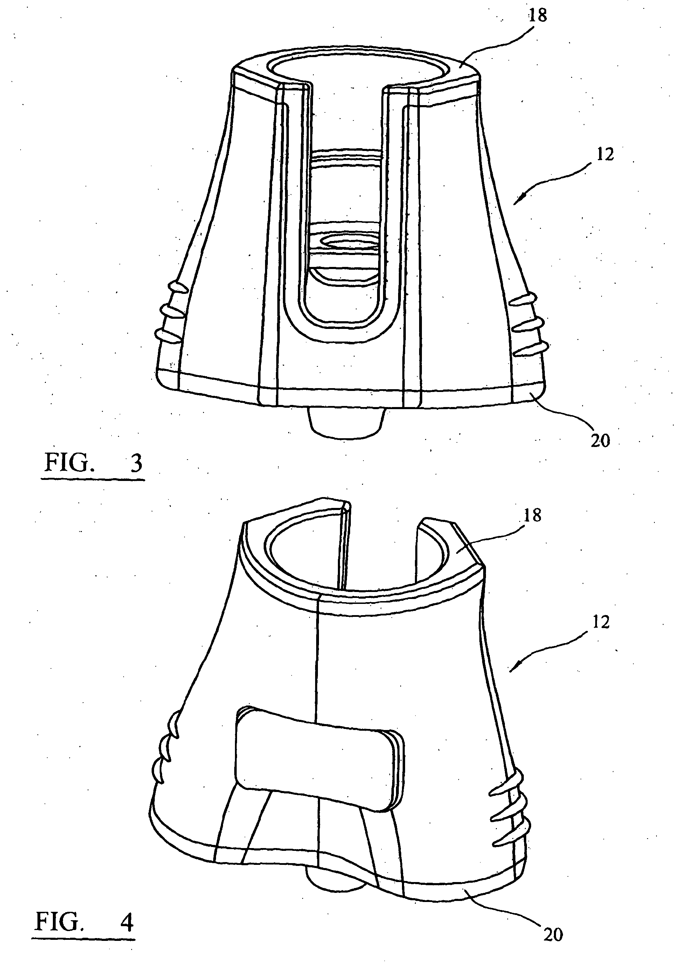

Referring to figures 1 to 4, these illustrate a two shot moulding method for manufacturing a surgical impactor in accordance with a first embodiment of the present invention. Figures 1 and 2 illustrate an interim component 10 formed through a first injection moulding step. Figures 3 and 4 illustrate a finished impactor 12 formed through a second injection moulding step, where part or the whole of the interim component 10 is present in the mould during the second injection moulding step. Figures 1 and 3 comprise first perspective views of the interim component or impactor and figures 2 and 4 comprise second, alternative perspective views of the interim component or impactor.

Figures 1 and 2 show that the shape of the mould in the first injection moulding step is chosen such that the interim component 10 comprises a series of walls and ribs, for instance ribs 14. Each wall or rib has a maximum thickness which is less than or equal to the maximum thickness achievable for an injection moulding step using the plastic material selected for the interim component 10. Channels, for instance channel 16, are defined between adjacent pairs of ribs. It can be seen that the ribs 14 substantially define the shape and size of the finished impactor 12.

It may be that the interim component 12 could have the required strength to use as an impactor, dependent upon the arrangement of walls and ribs, without further processing. However, it is clear that an impactor having numerous channels, as is the case for the interim component 10, would be difficult to clean, and therefore would be unacceptable in a medical environment.

By positioning the interim component 10 in a suitable mould, a second injection moulding step can be used to substantially or fully fill the channels 16 with plastic to form the finished impactor 12. The plastic material applied in the second injection moulding step may substantially or fully cover the interim component 12, or the second injection moulding step may be restricted to filling the channels 16. Either way, to ensure that the second injection moulding step does not result in surface sinks or voids, the maximum width of the channels 16 in the interim component is arranged to be less than or equal to the maximum thickness achievable for an injection moulding step using the plastic material selected for the second injection moulding step. Furthermore, for portions of the interim component 10 completely covered by the plastic applied in the second injection moulding step, the thickness of covering plastic is less than or equal to the same maximum thickness. It will be appreciated that the result of this is that the ribs forming the interim component 10 define the three dimensional shape of the finished component to within a limit set by the maximum thickness of the plastic injected in the second injection moulding step.

For the particular exemplary surgical instrument illustrated in figures 1 to 4 it can be seen that the interim component defines an upper and lower flanges 18, 20. The second injection moulding step may comprise inserting the interim component 10 into a mould which seals to the flanges 18, 20 such that the channels 16 are filled by plastic in the second injection moulding step between the flanges 18, 20.

The same plastic is used in the first and second injection moulding steps, in which case it may be difficult or impossible to discern from the finished product whether the product has been formed through two injection moulding steps. In such a situation it is clear that the maximum permissible thickness of the ribs and walls and the maximum width of the channels will be the same, as both are defined by the material properties of the same plastic material (though clearly, in practice, the ribs and channels may vary in dimensions up to those limits). By "similar" it is intended that the plastics used in each injection moulding step are generally the same, with similar chemical, structural or function properties. In particular embodiments of the present invention, where different but similar plastics are used, each plastic may be within the group of plastics known in the plastics industry as "high performance polymers" and "ultra high performance polymers" as these generally have high resistance to chemicals, moisture and temperature as well as high stiffness.

While embodiments of the present invention described above generally relate to surgical instruments, and methods of manufacturing such instruments, the same manufacturing techniques may also be applied to manufacturing surgical prostheses. In particular, the manufacturing techniques described above may be applied whenever there is a requirement to form a component with a thick section, which cannot be achieved in a single injection moulding step, and where there is a need to produce an easy to clean finished article. The above described techniques are particularly suited to applications where a finished article capable for bearing large loads is required. Advantageously the manufacturing cost may be reduced significantly compared with previously known alternative manufacturing techniques.

## Claims

1. A method of manufacturing a surgical instrument (12) or prosthesis comprising:
injecting a first material into a first mould to form an interim component (10), portions of the interim component defining at least one channel (16); and
injecting the second material into a second mould containing at least part of the channel of the interim component so that the channel is filled with the second material, **characterized in that**, the maximum wall thickness of the interim component being less than 5mm, and the maximum width of the at least one channel being less than 5mm, wherein the first material and the second material have similar structural properties and comprise the same plastic.

2. A method according to claim 1, wherein the interim component (10) comprises at least two ribs (14) each having a thickness less than 5mm, the at least one channel (16) being defined between the ribs.

3. A method according to claim 1 or 2, wherein the interim component and the second mould are arranged such that the maximum width of the cavity between the interim component and the mould wall is less than 5mm.

4. A method according to claim 1, wherein at least one of the first and second materials further comprise at least one additive.

5. A method according to any one of claims 1 to 4, wherein the plastic comprises a high or ultra high performance polymer.

6. The method of claim 1, wherein the method is a method of manufacturing an impactor.

7. An injection moulded surgical instrument (12) or prosthesis comprising:
a body;
wherein the body is formed in a two shot injection moulding process using a first material and a second material respectively the body comprising interlaced portions of said first and second materials **characterized in that** the body has a minimum thickness which exceeds 5mm and the maximum thickness of any portion of the first material is less than 5mm and the maximum thickness of any portion of the second material is less than 5mm and wherein the first material and the second material have similar structural properties and comprise the same plastic.

## Patentansprüche

1. Verfahren zur Herstellung eines chirurgischen Instruments (12) oder einer Prothese, aufweisend:
Injizieren eines ersten Materials in eine erste Form, um eine Zwischenkomponente (10) zu bilden, wobei Teile der Zwischenkomponente zumindest einen Kanal (16) definieren; und
Injizieren des zweiten Materials in eine zweite Form, die zumindest einen Teil des Kanals der Zwischenkomponente enthält, sodass der Kanal mit dem zweiten Material gefüllt ist, **dadurch gekennzeichnet, dass**
die maximale Wandstärke der Zwischenkomponente weniger als 5mm und die maximale Breite des zumindest einen Kanals weniger als 5mm beträgt, wobei das erste Material und das zweite Material ähnliche strukturelle Eigenschaften und denselben Kunststoff aufweisen.

2. Verfahren nach Anspruch 1, wobei die Zwischenkomponente (10) zumindest zwei Rippen (14) aufweist, die jeweils eine Breite von weniger als 5mm aufweisen, wobei der zumindest eine Kanal (16) zwischen den Rippen definiert ist.

3. Verfahren nach Anspruch 1 oder 2, wobei die Zwischenkomponente und die zweite Form so eingerichtet sind, dass die maximale Breite der Aushöhlung zwischen der Zwischenkomponente und der Wand der Form weniger als 5mm beträgt.

4. Verfahren nach Anspruch 1, wobei zumindest eines von dem ersten und dem zweiten Material weiter zumindest ein Additiv aufweist.

5. Verfahren nach irgendeinem der Ansprüche 1 bis 4, wobei der Kunststoff ein Hoch- oder Ultrahochleistungspolymer aufweist.

6. Verfahren nach Anspruch 1, wobei das Verfahren ein Verfahren zur Herstellung eines Impaktors ist.

7. Ein spritzgussgeformtes chirurgisches Instrument (12) oder eine Prothese, aufweisend:
einen Körper, wobei der Körper in einem Spritzgussprozess mit zwei Einspritzungen unter Verwendung eines ersten Materials bzw. eines zweiten Materials gebildet ist, wobei der Körper verschränkte Teile aus dem ersten und zweiten Material aufweist, **dadurch gekennzeichnet, dass** der Körper eine minimale Stärke aufweist, die 5mm übersteigt, und die maximale Stärke eine beliebigen Teils aus dem ersten Material weniger als 5mm und die maximale Stärke eines beliebigen Teils aus dem zweiten Material weniger als 5mm beträgt und wobei das erste Material und das zweite Material ähnliche strukturelle Eigenschaften und denselben Kunststoff aufweisen.

## Revendications

1. Procédé de fabrication d'un instrument chirurgical (12) ou d'une prothèse, comprenant :
injecter un premier matériau dans un premier moule pour former un composant intérimaire (10), des portions du composant intérimaire définissant au moins un canal (16) ; et
injecter le deuxième matériau dans un deuxième moule contenant au moins une partie du canal du composant intérimaire de sorte que le canal est rempli avec le deuxième matériau,
**caractérisé en ce que** l'épaisseur de paroi maximum du composant intérimaire est inférieure à 5 mm, et la largueur maximum dudit au moins un canal est inférieure à 5 mm ; où le premier matériau et le deuxième matériau ont des propriétés structurelles similaires et comprennent le même plastique.

2. Procédé selon la revendication 1, dans lequel le composant intérimaire (10) comprend au moins deux nervures (14) chacune d'une épaisseur inférieure à 5 mm, ledit au moins un canal (16) étant défini entre les nervures.

3. Procédé selon la revendication 1 ou 2, dans lequel le composant intérimaire et le deuxième moule sont agencés de telle sorte que la largueur maximum de la cavité entre le composant intérimaire et la paroi du moule est inférieure à 5 mm.

4. Procédé selon la revendication 1, dans lequel au moins un des premier et deuxième matériaux comprend en outre au moins un additif.

5. Procédé selon l'une quelconque des revendications 1 à 4, dans lequel le plastique comprend un polymère de performance élevée ou ultra-élevée.

6. Procédé selon la revendication 1, où le procédé est un procédé de fabrication d'un élément de frappe.

7. Instrument chirurgical moulé par injection (12) ou prothèse, comprenant :
un corps ;
où le corps est formé durant un processus de moulage par injection à deux charges utilisant un premier matériau et un deuxième matériau respectivement, le corps comprenant des portions entrelacées desdits premier et deuxième matériaux, **caractérisé en ce que** le corps a une épaisseur minimum qui dépasse 5mm et l'épaisseur maximum de n'importe quelle portion du premier matériau est inférieure à 5 mm, et l'épaisseur maximum de n'importe quelle portion du deuxième matériau est inférieure à 5 mm, et où le premier matériau et le deuxième matériau ont des propriétés structurelles similaires et comprennent le même plastique.
